# EUROPEAN PATENT SPECIFICATION

(11) **EP 2 606 896 B1**
(45) Date of publication and mention of the grant of the patent: **27.08.2014**
(21) Application number: 11195579.5
(22) Date of filing: 23.12.2011
(51) Int. Cl.: A61K 31/573, A61K 31/728, A61K 9/10, A61K 9/00, A61P 19/02

(54) **Sterile emulsion containing hyaluronate and glucocorticoid, and use of such emulsion in the treatment of inflammatory joint disorders**
Sterile Emulsion mit Hyaluronat und Glucocorticoid sowie Verwendung einer solchen Emulsion bei der Behandlung entzündlicher Gelenkerkrankungen
Émulsion stérile contenant de l'hyaluronate et glucocorticoïde et utilisation d'une telle émulsion pour le traitement des troubles articulaires inflammatoires

(43) Date of publication of application: 26.06.2013
(73) Proprietor: AAP Implantate AG, 12099 Berlin (DE)
(72) Inventor: Pelikaan, Hubert Clemens, 3572 CA Utrecht (NL); Dingeldein, Elvira, DE-63933 Mönchberg (DE)
(74) Representative: Nederlandsch Octrooibureau

(56) References cited:
- EP-A1- 0 913 149
- US-A1- 2008 044 476
- GRECOMORO G ET AL: "THERAPEUTIC SYNERGISM BETWEEN HYALURONIC ACID AND DEXAMETHASONE IN THE INTRA-ARTICULAR TREATMENT OF OSTEOARTHRITIS OF THE KNEE: A PRELIMINARY OPEN STUDY", CURRENT MEDICAL RESEARCH AND OPINION, HANTS, GB, vol. 13, no. 1, 1 January 1992 (1992-01-01), pages 49-55, XP009061294,
- CIHAT OZTURK ET AL: "The safety and efficacy of intraarticular hyaluronan with/without corticosteroid in knee osteoarthritis: 1-year, single-blind, randomized study", RHEUMATOLOGY INTERNATIONAL ; CLINICAL AND EXPERIMENTAL INVESTIGATIONS, SPRINGER, BERLIN, DE, vol. 26, no. 4, 1 February 2006 (2006-02-01), pages 314-319, XP019335312, ISSN: 1437-160X, DOI: 10.1007/S00296-005-0584-Z

## Description

### TECHNICAL FIELD OF THE INVENTION

The present invention relates to a sterile emulsion comprising a continuous aqueous phase containing hyaluronate and a dispersed lipid phase containing a glucocorticoid, such as dexamethasone.

The invention also relates to the use of such an emulsion in the treatment of inflammatory joint disorders, e.g. by intra-articularly administrating said emulsion.

### BACKROUND OF THE INVENTION

Arthritis is a form of joint disorder that involves inflammation of one or more joints. There are over 100 different forms of arthritis. The most common form, osteoarthritis (degenerative joint disease) is a result of trauma to the joint, infection of the joint, or age. Other arthritis forms are rheumatoid arthritis, psoriatic arthritis, and related autoimmune diseases. Septic arthritis is caused by joint infection.

The major complaint by individuals who have arthritis is joint pain. Pain is often a constant and may be localized to the joint affected. The pain from arthritis occurs due to inflammation that occurs around the joint, damage to the joint from disease, daily wear and tear of joint, muscle strains caused by forceful movements against stiff, painful joints and fatigue.

Most chronic joint disorders such as rheumatoid arthritis, psoriatic arthritis and osteoarthrosis are characterized by degradation of the structures in articular cartilage. Also acute inflammation of a joint is often accompanied by destruction of the cartilage, although in most cases this will not develop into the chronically destructive disease. It is not known which factors are crucial for the acutely inflamed joint to either proceed to healing or develop into the chronic process.

Presently, the primary method of treating rheumatoid arthritis is by use of orally ingested or otherwise systemically administered compounds directed at blocking the inflammatory process. These compounds include aspirin, penicillamine, gold salts, corticosteroids and many other drugs. Unfortunately, these attempts are often unsuccessful or associated with unacceptable side effects and the relief provided is temporary at best.

An alternative mode of treatment is to inject anti-inflammatory agents or substances which improve lubrication between surfaces of the joint directly into the synovial cavity of the afflicted joint, thus minimizing the risk of systemic complications.

An example of such an approach is the intra-articular injection of highly viscous or visco-elastic colloids such as high molecular weight hyaluronate (a natural constituent of the synovial fluid) or its cross-linked derivatives to cushion and lubricate apposing structures within the synovial cavity. Unfortunately this treatment is not always successful.

Grecomoro et al. (Curr Med Res Opin. (1992) 13(1): 49-55) report the result of a preliminary open study showing therapeutic synergism between hyaluronic acid and dexamethasone in the intra-articular treatment of osteoarthritis of the knee. Patients received a weekly intra-articular injection of 20 mg sodium hyaluronate and 0.4 mg dexamethasone phosphate in 2 ml phosphate buffer for 5 weeks.

Ozturk et al. (Thumatol. Int (2006) 26: 314-319) describe the results of study that was carried out to assess the safety and efficacy of hyaluronate with/without corticosteroid in patients with knee osteoarthritis. In a 1-year, randomized, single-blind trial, 24 patients were treated with hyaluronate weekly for 3 weeks, then three injections on the 6th month for a total of six injections. Sixteen patients were treated the same but with the addition of 1 ml triamcinolone acetonide (Kenacort-A) prior to the first and fourth hyaluronate injection.

US 2008/044476 describes viscous aqueous formulations useful for intramuscular and intra-articular administration. The Examples of this US patent application describe aqueous formulations containing triamcinolone acetonide (2-4% w/v) and sodium hyaluronate.

Thakker et al. (Drugs R D (2007) 8: 275-185 describes the outcome of a study that was conducted to determine whether the retention of celecoxib in inflamed articular joints of arthritic rats could be enhanced by incorporation of the drug into solid lipid nanoparticles. Celecoxib-loaded solid lipid nanoparticles (SLN) were prepared by emulsification and highpressure homogenisation. *In vitro* drug-release studies indicated that the nanoparticles exhibited sustained release of celecoxib. The biocompatibility of solid lipid nanoparticles was evaluated by histopathology of the rat joints after intra-articular injection in normal rats. Free celecoxib underwent rapid clearance from the inflamed articular joints into the systemic circulation, while the celecoxib-loaded SLN were associated with significantly lower blood levels compared with free celecoxib.

### SUMMARY OF THE INVENTION

The inventors have developed a novel formulation that can suitably be administered intra-articularly to patients suffering from an inflammatory joint disorder to maintain mobility of the joint and to provide effective relief for intra-articular pain and inflammation. The formulation of the present invention is a sterile emulsion comprising 80-99.9 wt.% of a continuous aqueous phase and 0.01-20 wt.% of a dispersed lipid phase, wherein the emulsion contains 0.1-10% of hyaluronate by weight of said aqueous phase; and 0.01-50% of a lipophilic glucocorticoid by weight of said lipid phase.

The emulsion according to the present invention offers the important advantage that following intra-articular administration it is effective for a significantly longer period of time than the formulations of the prior art.

Although the inventors do not wish to be bound by theory it is believed that the present emulsion is capable of reducing the inflamed state for a prolonged period of time. This effect includes a reduction of the flux of synovial fluid. This reduction can provide for a longer residence time of the injected hyaluronic acid as well as for a prolongation of the synthesis time of the endogenous hyaluronic acid. As a result, the endogeneous hyaluronic acid will have a higher molecular weight and exhibit improved lubrication and cushioning action. Thus, the emulsion makes it possible to maintain the viscosity of the synovial fluid at an acceptable level using intervals between intra-articular administrations that are substantially longer than those normally used in intra-articular treatment of arthritis with hyaluronate.

Furthermore, the present emulsion is also very effective when administered only once, e.g. in the treatment of acute inflammatory joint disorders, such as injury induced arthritis.

### DETAILED DESCRIPTION OF THE INVENTION

Accordingly, one aspect of the invention relates to a sterile emulsion comprising 80-99.9 wt.% of a continuous aqueous phase and 0.01-20 wt.% of a dispersed lipid phase, wherein the emulsion contains 0.1-10% of hyaluronate by weight of said aqueous phase; and 0.01-50% of a lipophilic glucocorticoid by weight of said lipid phase.

The term "hyaluronate" as used herein refers to an anionic, nonsulfated glycosaminoglycan distributed widely throughout connective, epithelial, and neural tissues. It is unique among glycosaminoglycans in that it is nonsulfated, forms in the plasma membrane instead of the Golgi, and can be very large, with its molecular weight often reaching the millions. The average 70 kg person has roughly 15 grams of hyaluronate in the body, one-third of which is turned over (degraded and synthesized) every day.

The term "glucocorticoid" as used herein refers to a class of steroid hormones that bind to the glucocorticoid receptor, which is present in almost every vertebrate animal cell. Glucocorticoids are part of the feedback mechanism in the immune system that turns immune activity (inflammation) down. They are therefore used in medicine to treat diseases that are caused by an overactive immune system, such as allergies, asthma, autoimmune diseases and sepsis.

The terminology "lipophilic glucocorticoid" as used herein refers to a glucortocoid that is readily soluble in triglyceride oil. Whenever reference is made herein to "% by weight" this should be construed as "% (w/w)".

Emulsifiers having an HLB of 6 or more are not considered to be part of the lipid phase.

The lipophilic glucocorticoid of the present invention preferably has a solubility in triolein at 20°C of at least 0.2g/l, more preferably of at least 2 g/l and most preferably of at least 10 g/l.

The lipophilic glucocorticoid typically has a water solubility of less than 2 g/l, more preferably of less than 1,5 g/l and most preferably of less than 1 g/l at 37°C.

Examples of lipophilic glucocorticoids that may suitably be used in the present emulsion include dexamethasone, betamethasone, triamcinolone, methylprednisolone, prednisone, hydrocortisone, cortisone and combinations thereof. Preferably, the glucorticoid is selected from dexamethasone, triamcinolone and combinations thereof. Most preferably, the glucocorticoid is dexamethasone.

The amount of glucorticoid in the present emulsion preferably lies within the range of 0.01-10 %, more preferably 0.02-5 % and most preferably 0.03-3 % by weight of the lipid phase. Expressed differently, the emulsion typically contains the glucorticoid in a concentration of 0.001-0.1%, more preferably of 0.003-0.08% and most preferably of 0.005-0.05% by weight of the total emulsion.

In accordance with a particularly preferred embodiment, the sterile emulsion contains the glucocorticoid in an amount that is equivalent to 0.01-1 mg/ml of dexamethasone, more preferably 0.03-0.8 mg/ml and most preferably 0.05-0.5 mg/ml of dexamethasone.

The dispersed lipid phase typically represents 0.5-20 wt.% of the present emulsion. More preferably, said lipid phase represents 1-18 wt.%, most preferably 1.5-15 wt.% of the emulsion.

The continuous aqueous phase and the dispersed lipid phase of the present emulsion together typically represent at least 99.5 wt.%, most preferably 100 wt.% of the emulsion.

The lipid phase of the sterile emulsion preferably contains at least 60 wt.%, more preferably at least 80 wt.% of one or more lipid components selected from triglycerides, diglycerides, monoglycerides, fatty acids, paraffin, mineral oils and combinations thereof. The aforementioned one or more lipid components are preferably selected from triglycerides, diglycerides, monoglycerides, fatty acids and combinations thereof.

In order to achieve a truly prolonged effect of intra-articular treatment with the present emulsion it is advisable that the one or more lipid components employed in the lipid phase have a combined melting point of at least 40°C, preferably of at least 45 °C. Typically, said one or more lipid components have a solid lipid content of at least 30 wt.%, more preferably of at least 40 wt.% at 37°C.

The prolonged effect of intra-articular treatment with the present emulsion may be extended by using a lipid phase consisting of relatively coarse droplets of lipid. Advantageously, the lipid phase has a volume weighted average droplet size of 3-50 µm. More preferably, the lipid phase has a volume weighted average droplet size of 5-40 µm, most preferably of 10-30 µm.

The lipid phase contained in the present emulsion preferably has a relatively narrow droplet size distribution. Accordingly, it is preferred that at least 80 wt.% of the lipid phase has a droplets size in the range of 3-50 µm, more preferably of 10-40 µm and most preferably of 10-30 µm.

In order to ensure that the emulsion remains stable during storage, the present emulsion advantageously contains 1-200 mg/m/, more preferably 3-100 mg/ml, most preferably 5-50 mg/ml of an emulsifier having an HLB value of at least 8, more preferably of at least 10, most preferably of at least 12.

Examples of emulsifiers that may suitably be employed in the present emulsion include polysorbate, polyethylene glycol and combination thereof. More preferably, the emulsifier employed in the present emulsion is polysorbate. Most preferably the emulsifier is selected from polysorbates comprising 15-90, preferably 20-80 oxyethylene -(CH₂CH₂O)-groups in the molecule.

The amount of hyaluronate contained in the present the emulsion preferably lies in the range of 0.02-5.0%, more preferably of 0.1-0.5% by weight of the aqueous phase.

The hyaluronate typically has a molecular weight in the range of 0.1-10 MDa. Most preferably, the hyaluronate has a molecular weight in the range of 0.5-7 MDa.

The emulsion of the present invention preferably is isotonic, having an osmolality of 200-350 mOsm/l.

It is further preferred that the emulsion has a viscosity of 10-10,000 cP at 37°C at a shear rate of 10 s⁻¹. Even more preferably the latter viscosity is at least 100 cP, most preferably at least 1,000 cP.

Another aspect of the invention relates to the use of the sterile emulsion as defined herein before in the treatment of inflammatory joint disorder in mammals, especially humans. Examples of inflammatory joint disorder that may suitably be treated with the present emulsion include rheumatoid arthritis, osteoarthritis and injury induced arthritis.

In accordance with a particularly preferred embodiment, the treatment of the inflammatory joint disorder comprises intra-articular administration of the sterile emulsion by injection. Most preferably, said treatment comprises administration of the emulsion to a knee.

The present invention offers the advantage that in the treatment of chronic inflammatory joint disorders the duration of the interval between different intra-articular administrations can be relatively long. Accordingly, it is preferred that in the present treatment the emulsion is administered at intervals of at least 7 days, more preferably of at least 10 days and most preferably of 15-30 days.

The present invention also provides an improved method for the treatment of an acute inflammatory joint disorder, e.g. treatment of injury induced arthritis, wherein the treatment comprises one time intra-articular administration of the present emulsion.

The treatment of inflammatory joint disorders in accordance with the present invention typically comprises administration of the sterile emulsion in a dose of 0.25-5 ml, most preferably of 1-3 ml.

The dose in which the sterile emulsion is administered in accordance with the present treatment preferably delivers 10-2,000 µg, more preferably 500-1,000 µg of glucocorticoid.

Expressed differently, the sterile emulsion is preferably administered in a dose that is equivalent to 10-2,000 µg dexamethasone, more preferably 500-1,000 µg dexamethasone.

The invention is further illustrated by means of the following non-limiting examples.

### EXAMPLES

### Example 1

Emulsions were prepared on the basis of the formulations shown in Table 1.

**Table 1**

| | 1 | 2 | 3 | Control |
|---|---|---|---|---|
| Tween 20 | 16.0 | 16.0 | 16.0 | 16.0 |
| Myglyol® 812 ^{#} | 10.0 | | | |
| Witepsol® H15^{$} | | 10.0 | | |
| Decanoic acid | | | 10.0 | |
| Dexamethasone | 0.01 | 0.01 | 0.01 | 0.01 |
| Hynoval* | Remainder | | | |

| | | | | |
|---|---|---|---|---|
| ^{#} Caprylic/Capric Triglyceride (Sasol Germany GmbH) ^{$} Hydrogenated coconut oil, melting point ±35°C (Sasol Germany GmbH) ^{*} A sterile, solution (10 mg/ml) of high MW sodium hyaluronate (Synovamed AG) | | | | |

The emulsions (25 ml) were prepared by:
1) dissolving dexamethasone in the lipid phase component,
2) adding Tween 20 to the hyaluronate solution,
3) adding the lipid phase component with dexamethasone to the hyaluronate solution,
4) setting a temperature of 50 °C,
5) mixing the components with an Ultra-Turrax® for 60 seconds,
6) cooling the emulsion down to 20 °C while turraxing.

The volume weighted diameter of the dispersed lipid phase of Emulsions 1-3 was found to be approximately 20 µm.

The release characteristics of these emulsions were tested using the following procedure: 2 ml of the described formulations are brought into a dialysis tube. These dialysis tubes are suspended into closable tubes filled with 25 ml simulated body fluid (SBF) buffered at pH 7.0. These tubes are kept at 37 °C. Each 48 hours this fluid is sampled for analysis and refreshed with new SBF. The results obtained from these tests are summarized in Table 2.

**Table 2**

| Emulsion | Dexamethasone conc. <0.01 µg/ml after |
|---|---|
| 1 | 9 days |
| 2 | ➢ 10 days |
| 3 | 8-9 days |
| Control | < 6 days |

## Claims

1. A sterile emulsion comprising 80-99.9 wt.% of a continuous aqueous phase; and 0.01-20 wt.% of a dispersed lipid phase, wherein the emulsion contains 0.1-10% of hyaluronate by weight of said aqueous phase; and 0.01-50% of lipophilic glucocorticoid by weight of said lipid phase.

2. Sterile emulsion according to claim 1, wherein the glucocorticoid is selected from dexamethasone, betamethasone, triamcinolone, methylprednisolone, prednisone, hydrocortisone, cortisone and combinations thereof.

3. Sterile emulsion according to claim 1 or 2, wherein the sterile emulsion contains the glucocorticoid in an amount that is equivalent to 0.1-10 mg/ml of dexamethasone.

4. Sterile emulsion according to any one of the preceding claims, wherein the one or more lipid components have a combined melting point of at least 40°C.

5. Sterile emulsion according to any one of the preceding claims, wherein the lipid phase has a volume weighted average droplet size of 3-50 µm.

6. Sterile emulsion according to any one of the preceding claims, wherein the continuous aqueous phase and the dispersed lipid phase together represent at least 99.5 wt.%, most preferably 100 wt.% of the emulsion.

7. Sterile emulsion according to any one of the preceding claims, wherein the emulsion contains 1-200 mg/ml of an emulsifier having an HLB value of at least 6.

8. Sterile emulsion according to any one of the preceding claims, wherein the emulsion contains 0.02-5.0% of hyaluronate by weight of the aqueous phase.

9. Sterile emulsion according to any one of the preceding claims, wherein the hyaluronate has a molecular weight in the range of 0.1-10 MDa.

10. Sterile emulsion according to any one of the preceding claims, wherein the emulsion has a viscosity of 10-10,000 cP at 37°C at a shear rate of 10 s⁻¹.

11. Sterile emulsion according to any one of the preceding claims for use in the treatment of inflammatory joint disorder in mammals.

12. Sterile emulsion for use according to claim 11, wherein the inflammatory joint disorder is selected from rheumatoid arthritis, osteoarthritis and injury induced arthritis.

13. Sterile emulsion for use according to claim 10 or 11, wherein the sterile emulsion is administered intra-articularly.

14. Sterile emulsion for use according to claim 13, wherein the emulsion is administered at intervals of at least 7 days.

15. Sterile emulsion for use according to any one of claims 11-14, wherein the emulsion is administered in a dose that is equivalent to 500-2,000 µg dexamethasone.

## Patentansprüche

1. Eine sterile Emulsion umfassend 80 bis 99,9 Gewichtsprozent einer kontinuierlichen wässrigen Phase und 0,01 bis 20 Gewichtsprozent einer dispergierten Lipidphase, wobei die Emulsion 0,1 bis 10 Prozent Hyaluronat bezogen auf das Gewicht der wässrigen Phase und 0,01 bis 50 Prozent lipophiles Glucocorticoid bezogen auf das Gewicht der Lipidphase enthält.

2. Sterile Emulsion gemäß Anspruch 1, wobei das Glucocorticoid aus Dexamethason, Betamethason, Triamcinolon, Methylprednisolon, Prednison, Hydrocortison, Cortison und Kombinationen von diesen ausgewählt ist.

3. Sterile Emulsion gemäß Anspruch 1 oder 2, wobei die sterile Emulsion das Glucocorticoid in einer Menge enthält, die äquivalent zu 0,1 bis 10 mg/ml Dexamethason ist.

4. Sterile Emulsion gemäß irgendeinem der vorhergehenden Ansprüche, wobei die eine Lipidkomponente oder die mehreren Lipidkomponenten einen kombinierten Schmelzpunkt von wenigstens 40° C haben.

5. Sterile Emulsion gemäß irgendeinem der vorhergehenden Ansprüche, wobei die Lipidphase eine volumengewichtete Durchschnittströpfchengröße von 3 bis 50 □m hat.

6. Sterile Emulsion gemäß irgendeinem der vorhergehenden Ansprüche, wobei die kontinuierliche wässrige Phase und die dispergierte Lipidphase zusammen wenigstens 99,5 Gewichtsprozent, besonders bevorzugt 100 Gewichtsprozent der Emulsion darstellen.

7. Sterile Emulsion gemäß irgendeinem der vorhergehenden Ansprüche, wobei die Emulsion 1 bis 200 mg/ml eines Emulgators enthält, der einen HLB Wert von wenigstens 6 hat.

8. Sterile Emulsion gemäß irgendeinem der vorhergehenden Ansprüche, wobei die Emulsion 0,02 bis 5,0 Prozent Hyaluronat bezogen auf das Gewicht der wässrige Phase enthält.

9. Sterile Emulsion gemäß irgendeinem der vorhergehenden Ansprüche, wobei das Hyaluronat ein Molekulargewicht im Bereich von 0,1 bis 10 MDa hat.

10. Sterile Emulsion gemäß irgendeinem der vorhergehenden Ansprüche, wobei die Emulsion eine Viskosität von 10 bis 10.000 cP bei 37° C bei einer Schergeschwindigkeit von 10 s⁻¹ hat.

11. Sterile Emulsion gemäß irgendeinem der vorhergehenden Ansprüche zur Verwendung in einer Behandlung von einer entzündlichen Gelenkerkrankung von Säugetieren.

12. Sterile Emulsion zur Verwendung gemäß Anspruch 11, wobei die entzündliche Gelenkerkrankung aus rheumatoider Arthritis, Arthrose und verletzungsbedingter Arthritis ausgewählt ist.

13. Sterile Emulsion zur Verwendung gemäß Anspruch 10 oder 11, wobei die sterile Emulsion intraartikulär verabreicht wird.

14. Sterile Emulsion zur Verwendung gemäß Anspruch 13, wobei die Emulsion in Intervallen von wenigstens 7 Tagen verabreicht wird.

15. Sterile Emulsion zur Verwendung gemäß irgendeinem der Ansprüche 11 bis 14, wobei die Emulsion in einer Dosierung verabreicht wird, die äquivalent zu 500 bis 2.000 □g Dexamethason ist.

## Revendications

1. Émulsion stérile comprenant de 80 à 99,9% en poids d'une phase aqueuse continue ; et de 0,01 à 20% en poids d'une phase lipidique dispersée, dans laquelle l'émulsion contient de 0,1 à 10% d'hyaluronate en poids de ladite phase aqueuse ; et de 0,01 à 50% de glucocorticoïde lipophile en poids de ladite phase lipidique.

2. Émulsion stérile selon la revendication 1, dans laquelle le glucocorticoïde est choisi parmi la dexaméthasone, la bêtaméthasone, la triamcinolone, la méthylprednisolone, la prednisone, l'hydrocortisone, la cortisone et des combinaisons de celles-ci.

3. Émulsion stérile selon la revendication 1 ou 2, dans laquelle l'émulsion stérile contient le glucocorticoïde en une quantité qui est équivalente à une quantité allant de 0,1 à 10 mg/ml de dexaméthasone.

4. Émulsion stérile selon l'une quelconque des revendications précédentes, dans laquelle le ou les plusieurs composant(s) lipidique(s) a/ont un point de fusion combiné d'au moins 40°C.

5. Émulsion stérile selon l'une quelconque des revendications précédentes, dans laquelle la phase lipidique a une taille de gouttelettes moyenne pondérée en volume allant de 3 à 50 µm

6. Émulsion stérile selon l'une quelconque des revendications précédentes, dans laquelle la phase aqueuse continue et la phase lipidique dispersée représentent ensemble au moins 99,5% en poids, de manière la plus préférable 100% en poids de l'émulsion.

7. Émulsion stérile selon l'une quelconque des revendications précédentes, dans laquelle l'émulsion contient de 1 à 200 mg/ml d'un émulsifiant ayant une valeur HLB d'au moins 6.

8. Émulsion stérile selon l'une quelconque des revendications précédentes, dans laquelle l'émulsion contient de 0,02 à 5,0% d'hyaluronate en poids de la phase aqueuse.

9. Émulsion stérile selon l'une quelconque des revendications précédentes, dans laquelle l'hyaluronate a un poids moléculaire se trouvant dans la plage allant de 0,1 à 10 MDa.

10. Émulsion stérile selon l'une quelconque des revendications précédentes, dans laquelle l'émulsion a une viscosité allant de 10 à 10000 cP à 37°C à une vitesse de cisaillement de 10 s⁻¹.

11. Émulsion stérile selon l'une quelconque des revendications précédentes pour une utilisation dans le traitement de trouble articulaire inflammatoire chez les mammifères.

12. Émulsion stérile pour une utilisation selon la revendication 11, dans laquelle le trouble articulaire inflammatoire est choisi parmi la polyarthrite rhumatoïde, l'ostéoarthrite et l'arthrite induite par une lésion.

13. Émulsion stérile pour une utilisation selon la revendication 10 ou 11, dans laquelle l'émulsion stérile est administrée par voie intra-articulaire.

14. Émulsion stérile pour une utilisation selon la revendication 13, dans laquelle l'émulsion est administrée à des intervalles d'au moins 7 jours.

15. Émulsion stérile pour une utilisation selon l'une quelconque des revendications 11 à 14, dans laquelle l'émulsion est administrée à une dose qui est équivalente à une dose allant de 500 à 2000 µg de dexaméthasone.
